# EUROPEAN PATENT SPECIFICATION

(11) **EP 4 164 584 B1**
(45) Date of publication and mention of the grant of the patent: **04.11.2026**
(21) Application number: 21720226.6
(22) Date of filing: 16.04.2021
(51) Int. Cl.: A61K 8/03, A61K 8/34, A61K 8/58, A61Q 19/00

(54) **PERSONAL CARE COMPOSITION WITH VISUALLY DISTINCT AQUEOUS AND OIL PHASE**
KÖRPERPFLEGEZUSAMMENSETZUNG MIT EINER WÄSSRIGEN PHASE UND EINER FETTPHASE, DIE VISUELL UNTERSCHEIDBAR SIND
COMPOSITION POUR SOINS PERSONNELS COMPRENANT UNE PHASE AQUEUSE ET UNE PHASE GRASSE QUI SONT VISUELLEMENT DISTINCTES L'UNE DE L'AUTRE

(30) Priority: 09.05.2020 WO PCT/CN2020/089333; 18.06.2020 EP 20180664
(43) Date of publication of application: 19.04.2023
(73) Proprietor: Unilever IP Holdings B.V., 6708 WH Wageningen (NL); Unilever Global IP Limited, Wirral, Merseyside CH62 4ZD (GB)
(72) Inventor: BIAN, Wei, Shanghai, 200335 (CN); LIU, Lanhua, Shanghai, 200335 (CN); ZHANG, Yang, Shanghai, 200335 (CN)
(74) Representative: Unilever Patent Group
(86) International application number: PCT/EP2021/059985
(87) International publication number: WO 2021/228492

(56) References cited:
- EP-A1- 1 676 560
- JP-A- H01 294 615
- US-A1- 2007 009 446
- US-A1- 2019 142 706

## Description

### Field of the invention

The present invention relates to a multi-phase personal care composition. In particular, the present invention relates to a multi-phase personal care composition comprising an aqueous phase comprising an alcohol being an diol; and an oil phase comprising volatile silicone, wherein the aqueous phase is visually distinct from and in physical contact with the oil phase, the amount of the alcohol is 2 to 40% by weight of the composition and the weight ratio of the aqueous phase to the oil phase is in the range of 1:4 to 4:1, the composition comprises alkyl glucoside, and the composition is substantially free of polysorbate 20, wherein "Substantially free of" refers to the amount is 0 to 0.1 % by weight of the composition.

### Background of the invention

Nowadays, the personal care compositions with more user involvement are seen as more attractive by the consumer. The multi-phase personal care composition with rapid *in situ* generation of dispersion is an excellent example. The product delivered to the consumer is a multi-phase personal care composition with at least two visually distinct phases. Before applying, the consumer may generate a well-dispersed personal care product by shaking the container for a few seconds by hand. Then, the person care product is left to stand to recover to be multi-phase personal care composition before next use. It would be unpleasant if the consumer would like to use after one night and found that it has not been back to the multi-phase state. Therefore, how long it will take to recover to the multi-phase state is critical for user experience. CN111000735 relates a double-layer makeup remover which defines the aqueous/oil phases ratio as being EP1676560 concerns compositions consisting of two distinct phases, an aqueous phase and an emulsion, these phases mixing easily when agitated and separating easily when agitation ceases.

When the present inventors developed a multi-phase personal care composition containing volatile silicone, unexpectedly it was found that it took long time, for example overnight, for the product to recover to be a multi-phase personal care composition. It would be much less attractive to the consumer if it is not recovered yet when the consumers use the product again.

There is a need, therefore, for a way to develop a solution to improve the recovering time for the personal care product. It was surprisingly found that the recovering time was significantly shortened by incorporating alcohol having less than three hydroxyl groups into the aqueous phase of the multi-phase personal care composition.

### Summary of the invention

In a first aspect, the present invention is directed to a multi-phase personal care composition comprising an aqueous phase comprising an alcohol being a diol; and an oil phase comprising volatile silicone, wherein the aqueous phase is visually distinct from and in physical contact with the oil phase, the amount of the alcohol is 2 to 40% by weight of the composition and the weight ratio of the aqueous phase to the oil phase is in the range of 1:4 to 4:1, the composition comprises alkyl glucoside, and the composition is substantially free of polysorbate 20, wherein "Substantially free of" refers to the amount is 0 to 0.1 % by weight of the composition. "Substantially free of" refers to the amount is 0 to 0.1 % by weight of the composition.

In a second aspect, the present invention is directed to kit of parts comprises a cosmetic container, a multi-phase personal care composition of the present invention and instruction for use of the kit.

In a third aspect, the present invention is directed to a method for preparing a personal care product comprising a step of shaking a cosmetic container containing a multi-phase personal care composition of the present invention by human hand.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about". All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

"Visually distinct" as used herein refers the regions occupied by each phase can be separately seen by human eye as distinctly separate regions in contact with one another (i.e., they are not emulsions or dispersions of particles of less than 100 microns).

"Multi-phase" as used herein refers to that at least two phases occupy separate and distinct physical spaces inside the container in which they are stored, but are in direct contact with one another (i.e., they are not separated by a barrier).

"Transparent" as used herein refers to that at least 70%, preferably at least 80%, more preferably at least 85% of light, having wavelength of 550 nm transmits a 1 cm thick of sample, measured by a UV-vis spectrometer (e.g. Perkin-Elmer Lambda 650S) at 25 °C. "Opaque" as used herein refers to that no greater than 50%, preferably no greater than 30% of light transmits by same method.

"Substantially free of" refers to the amount is 0 to 0.1%, preferably less than 0 to 0.01%, and more preferably 0% by weight of the composition.

Preferably, the alcohol has 2 to 10 carbon atoms, more preferably 2 to 6 carbon atoms, and most preferably 2 to 4 carbon atoms. Preferably, the alcohol is water miscible.. It is preferable that the diol has from 2 to 10 carbon atoms, more preferably 3 to 6 carbon atoms, most preferably the diol has 3 to 4 carbon atoms.

Particularly preferred alcohol comprises polyethylene glycol, polypropylene glycol, diethylene glycol, dipropylene glycol, triethylene glycol, hexylene glycol, butylene glycol or a mixture thereof. Even more preferably, the alcohol comprises polypropylene glycol, butylene glycol, or a mixture thereof. Still even more preferably the alcohol comprises butylene glycol and most preferably the alcohol is butylene glycol. Preferably, the composition is substantially free of glycerin.

Preferably the amount of alcohol is 6 to 30% and more preferably from 12 and 18% by total weight of the composition.

The term "volatile silicone" is understood to mean a liquid polyorganosiloxane having a measurable vapor pressure at 25 °C and atmospheric pressure, preferably ranging from 0.13 Pa to 40 000 Pa, more preferably ranging from 1.3 Pa to 5 000 Pa and most preferably ranging from 10 Pa to 1300 Pa.

Preferably, the volatile silicone contains 2 to 15, more preferably 3 to 10 silicon atoms. Volatile silicone can be linear or cyclic or mixtures thereof. Preferred cyclic silicone include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms.

The viscosity of volatile silicone is typically from 0.01 to 100 cSt (centi-Stokes) at 25 °C, preferably from 0.1 cSt to 20 cSt, more preferably from 0.2 to 10 cSt.

The volatile silicone is preferably cyclopentasiloxane. The volatile silicone is preferably selected from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane, or a mixture thereof. More preferably, the volatile silicone is selected from decamethylcyclopentasiloxane, dodecamethylcyclohexasiloxane, or a mixture thereof. Most preferably the volatile silicone is decamethylcyclopentasiloxane.

Examples of commercially available silicone include silicone having grade designations 344, 345, 244, 245 and 246 from Dow Corning Corporation; Silicone 7207 and Silicone 7158 from Union Carbide Corporation; and SF1202 from General Electric

The weight ratio of the volatile silicone to the alcohol is preferably 50:1 to 1:10, more preferably 20:1 to 1:3 and most preferably 6:1 to 1:1.

Preferably, the composition comprises a non-ionic surfactant. Preferably, the non-ionic surfactant comprises: a) the condensation products of aliphatic alcohols having from 8 to 22 carbon atoms in either straight or branched chain configuration with ethylene oxide, such as a coconut alcohol/ethylene oxide condensates having from 2 to 15 moles of ethylene oxide per mole of coconut alcohol; b) condensates of alkylphenols having C6-C15 alkyl groups with 5 to 25 moles of ethylene oxide per mole of alkylphenol; c) alkyl glucoside, or a mixture thereof. More preferably the nonionic surfactant is alkyl glucoside. Alkyl glucoside as used herein includes alkyl polyglucosides.

Preferred alkyl glucoside is represented by formula of RO - (G)ₙ, wherein R is a branched or straight chain alkyl group which may be saturated or unsaturated, G is a saccharide group, and the degree of polymerisation, n, may have a value of from 1 to 10; Preferably R has a mean alkyl chain length of from C₅ to C₂₀, G is selected from C₅ or C₆ monosaccharide residues and n has a value of from 1 to 6; more preferably R has a mean alkyl chain length of from C₆ to C₁₆, G is glucose and n has a value of from 1 to 6.

Preferably, the amount of the non-ionic surfactant is 0.0001 to 15%, more preferably 0.001 to 10%, even more preferably 0.01 to 6%, still even more preferably 0.1 to 5%, and most preferably 1 to 4% by weight of the composition.

Preferably the composition is substantially free of polyoxyethylene sorbitan C₆-C₂₄ fatty acid ester and more preferably the composition is substantially free of polyoxyethylene sorbitan fatty acid esters. Preferably alkyl glucosides account for 90 to 100% of the total amount of nonionic surfactant in the composition. More preferably all nonionic surfactants in the composition are alkyl glucosides. Even more preferably, all nonionic surfactants in the composition are caprylyl/capryl glucoside.

Preferably, the composition comprises retinoid. Typically, the retinoid is selected from retinyl ester, retinol, retinal, retinoic acid or a mixture thereof. More preferably the retinoid comprises retinol, retinyl ester, or a mixture thereof and even more preferably the retinoid is selected from retinol, retinyl ester, or a mixture thereof.

The term "retinol" includes the following isomers of retinol: all-trans-retinol, 13-cis-retinol, 11-cis-retinol, 9-cis-retinol, 3,4-didehydro-retinol, 3,4-didehydro-13-cis-retinol; 3,4-didehydro-11-cis-retinol; 3,4-didehydro-9-cis-retinol. Preferred isomers are all-trans-retinol, 13-cis-retinol, 3,4-didehydro-retinol, 9-cis-retinol. Most preferred retinol is all-trans-retinol, due to its wide commercial availability. Retinyl ester is an ester of retinol. The term "retinol" has been defined above. Retinyl esters suitable for use in the present invention are preferably C₁-C₃₀ esters of retinol, more preferably C₂-C₂₀ esters of retinol, and most preferably C₂, C₃, and C₁₆ esters of retinol. The retinyl ester for use in the present invention is preferably selected from retinyl palmitate, retinyl acetate, retinyl linoleate, retinyl oleate, retinyl propionate or a mixture thereof. More preferably the retinyl ester is selected from retinyl palmitate, retinyl acetate, retinyl propionate, or a mixture thereof. Most preferably the retinyl ester is selected from retinyl palmitate, retinyl propionate, or a mixture thereof.

Particularly preferred retinoid is selected from all-trans-retinol, retinyl palmitate, retinyl acetate, retinyl propionate, or a mixture thereof. Most preferably the retinoid is selected from retinyl palmitate, retinyl propionate, or a mixture thereof.

Preferably, retinoid is employed in the composition in an amount of 0.0001 to 8% by weight of the composition, more preferably in an amount of 0.0005 to 3%, even more preferably from 0.1 to 1% and most preferably in an amount of 0.2 to 0.8% by weight of the composition.

Preferably the composition comprises fatty acid amide. Preferably, the fatty acid amide contains at least 6 carbon atoms. Suitable fatty acids include saturated and unsaturated, straight or branched fatty acids. Suitable fatty acids preferably contain from 8 to 24 carbon atoms, preferably from 12 to 20 carbon atoms, and most preferably from 12 to 18 carbon atoms, because longer chain fatty acid amides are more beneficial for conditioning of the skin. In the most preferred embodiment of the invention, amides of essential fatty acids are employed because essential fatty acids provide nutrition for the skin. Examples of essential fatty acids include but are not limited to linoleic, linolenic, arachidonic, gamma-linolenic, homo-gamma-linolenic, and mixtures thereof. Linoleic acid is most preferred because it is also a precursor to ceramide.

Amides suitable for use in the present invention may be simple amides (i.e., those containing a-CONH₂ group), N-alkyl amides, N, N-dialkyl amides, mono-alkanol amides, and di-alkanol amides. Suitable alkyl or alkanol groups contain from 1 to 30 carbon atoms, preferably from 1 to 20 carbon atoms, and most preferably from 1 to 8 carbon atoms. The preferred amides included in the present invention are mono- and di-alkanol amides, particularly of essential fatty acids. Alkanol amides are more commonly available than alkyl amides.

The preferred fatty acid amides are selected from mono- and diethanolamides of linoleic acid, palmitic acid, and coconut oil.

The fatty acid amide may be included in the inventive compositions in an amount ranging from 0.0001% to 10%, preferably from 0.01% to 4%, most preferably from 0.1% to 1% by weight of the total amount of the composition.

Preferably, the composition comprises triglyceride. More preferably, the composition comprises caprylic/capric triglyceride, coconut oil, sunflower seed oil, safflower oil, cottonseed oil, olive oil or a mixture thereof. Particularly preferred triglyceride is caprylic/capric triglyceride. Preferably, the amount of triglyceride is 0.001 to 12%, more preferably 0.1 to 8%, even more preferably 0.5 to 5% by weight of the composition.

The composition may comprise water in amount of 10 to 90% by weight of the composition, more preferably from 15 to 78%, even more preferably from 20 to 65%, most preferably from 25 to 50% by weight of the composition.

The composition may comprise optional ingredients including moisturizing agent, skin lightening agent, preservatives, antioxidants, colorants, fragrance, or a combination thereof.

Vitamin B3 compounds (including derivatives of vitamin B3) e.g. niacin, nicotinic acid or niacinamide are the preferred skin lightening agent as per the invention, most preferred being niacinamide.

Preferably, the composition is a bi-phase composition. Preferably, both the aqueous and oil phases are transparent. Preferably the weight ratio of the aqueous phase to the oil phase is preferably in the range of 1:8 to 8:1, more preferably 1:4 to 4:1, and even more preferably 1:2 to 2:1.

The multi-phase personal care composition can be provided to a consumer in any suitable way, but it is preferable that the composition is provided inside a cosmetics container. The cosmetics container preferably has a volume of 2 to 250 mL, more preferably 5 to 100 mL, even more preferably 8 to 60 mL and still even more preferably 10 to 50 mL.

Preferably a kit of parts comprises a cosmetic container, a multi-phase personal care composition according to the invention and instruction for use of the kit. Preferably, the instruction comprises the step of mixing aqueous phase with the oil phase by any suitable way, for example by shaking the container by human hand for at least 1 seconds, preferably 2 seconds to 5 minutes, more preferably 3 seconds to 1 minutes. Thus, a homogeneous personal care product is formed. Preferably the personal care product is opaque.

Preferably, the multi-phase personal care composition is capable of generating a personal care product by shaking the cosmetic container by human hand, preferably for 1 second to 5 minutes, more preferably 1 second to 1 minutes. Preferably, the personal care product is capable of recovering to be a multi-phase personal care composition by standing for 3 minutes to 20 hours, more preferably 10 minutes to 7 hours, and even more preferably 15 minutes to 4 hours.

Preferably, the personal care composition (product) is a skin care composition (product). The skin care composition (product) refers to a composition (product) suitable for topical application to human skin, preferably is a leave-on product. The term "leave-on" as used with reference to compositions herein means a composition that is applied to or rubbed on the skin, and left thereon. The term "skin" as used herein includes the skin on the face (except eye lids and lips), neck, chest, abdomen, back, arms, under arms, hands, and legs. Preferably "skin" means includes the skin on the face (except eye lids and lips) and under arms, more preferably skin means skin on the face other than lips and eyelids.

The following examples are provided to facilitate an understanding of the invention. The examples are not intended to limit the scope of the claims.

### Examples

### Example 1

This example demonstrates the effect of the solvent on the performance of the multi-phase skin care composition.

**Table 1**

| Phase | Ingredient | Samples (wt%)* | | | | |
|---|---|---|---|---|---|---|
| | | A | 1 | 2 | 3 | 4 |
| Aqueous | Water | To 100 | To 100 | To 100 | To 100 | To 100 |
| | Glycerin | 15.00 | - | - | 7.50 | 6.00 |
| | Butylene glycol | - | 15.00 | - | 7.50 | 9.00 |
| | Propylene glycol | - | - | 15.00 | - | - |
| | Cocamide MEA | 0.25 | 0.25 | 0.25 | 0.25 | 0.25 |
| | Caprylyl/Capryl Glucoside | 1.24 | 1.24 | 1.24 | 1.24 | 1.24 |
| | Phenoxyethanol | 0.60 | 0.60 | 0.60 | 0.60 | 0.60 |
| | Sodium Chloride | 0.60 | 0.60 | 0.60 | 0.60 | 0.60 |
| Oil | Cyclopentasiloxane | 39.67 | 39.67 | 39.67 | 39.67 | 39.67 |
| | Retinol Propionate | 0.33 | 0.33 | 0.33 | 0.33 | 0.33 |

| | | | | | | |
|---|---|---|---|---|---|---|
| *The level of the ingredients refers the level of active. | | | | | | |

The samples in Table 1 were prepared by mixing Cocamide MEA at a temperature of 50 to 60 °C, cooling down to room temperature, and adding other ingredients to get the aqueous phase. The ingredients for oil phase were mixed together to get the oil phase. The sample were obtained by mixing the aqueous phase and oil phase.

Then, each sample were packaged into three identical transparent jars with equal amount. All samples are colorless and transparent. All jars were shaken by human hand for 15 seconds at the same time. 5 uniformly dispersed skin care products were formed. They are let sit to recover to be the multi-phase state. The time for each sample for recovery to be multi-phase was recorded and the performance was described in Table 2.

**Table 2.**

| Sample | Time to recover |
|---|---|
| A | Not fully recovered after 24 hours |
| 1 | About 1 hour |
| 2 | About 2 hours |
| 3 | About 4 hours |
| 4 | About 4 hours |

It is surprisingly found that the recovering time is significantly shortened by including alcohol having less than three hydroxyl groups into the aqueous phase of the multi-phase personal care composition

### Example 2

This example demonstrates the effect of the surfactant on the performance of the multi-phase skin care composition.

**Table 3**

| Phase | Ingredient | Samples (wt%)* | |
|---|---|---|---|
| | | B | 5 |
| Aqueous | Water | To 100 | To 100 |
| | Butylene glycol | 15.00 | 15.00 |
| | Cocamide MEA | 0.25 | 0.25 |
| | Caprylyl/Capryl Glucoside | 1.24 | 1.55 |
| | Polysorbate 20 | 0.20 | - |
| | Phenoxyethanol | 0.60 | 0.60 |
| | Sodium Chloride | 0.60 | 0.60 |
| Oil | Decamethylcyclopentasiloxane | 39.67 | 39.67 |
| | Retinol Propionate | 0.33 | 0.33 |

| | | | |
|---|---|---|---|
| *The level of the ingredients refers the level of active. | | | |

The samples in Table 3 were prepared by following the identical procedures as described in Example 1. Same test was carried out as in Examples 1. The time to recover to be the multi-phase state for samples B and 5 to be multi-phase are about 10 hours and 0.5 hours respectively.

It was surprisingly found that with addition of polysorbate 20, the time to recover was significantly increased. In contrast, the composition is capable of being recovered to multi-phase state when all nonionic surfactants are alkyl glucoside.

## Claims

1. A multi-phase personal care composition comprising:
(a) an aqueous phase comprising an alcohol being diol; and
(b) an oil phase comprising volatile silicone,
wherein:
i) the aqueous phase is visually distinct from and in physical contact with the oil phase,
ii) the amount of the alcohol is 2 to 40% by weight of the composition,
iii) the weight ratio of the aqueous phase to the oil phase is in the range of 1:4 to 4:1,
iv) the composition comprises alkyl glucoside, and
v) the composition is substantially free of polysorbate 20, wherein "Substantially free of" refers to the amount is 0 to 0.1% by weight of the composition.

2. The composition according to claim 1 wherein the alcohol has 2 to 10 carbon atoms, preferably the alcohol comprises butylene glycol.

3. The composition according to claim 1 or 2 wherein the amount of the alcohol is 6 to 30% by weight of the composition.

4. The composition according to any one of the preceding claims wherein the volatile silicone contains 3 to 10 silicon atoms, preferably the volatile silicone is decamethylcyclopentasiloxane.

5. The composition according to any one of preceding claims wherein the amount of the volatile silicone is in the range of 3 to 60%, preferably from 28 to 42% by weight of the total amount of the composition.

6. The composition according to any one of the preceding claims wherein the weight ratio of the volatile silicone to the alcohol is 50:1 to 1:10, preferably 6:1 to 1:1.

7. The composition according to any one of the preceding claims wherein the composition comprises a retinoid, preferably retinyl palmitate, retinyl propionate, or a mixture thereof.

8. The composition according to any one of the preceding claims wherein both the aqueous phase and oil phase are transparent.

9. The composition according to any one of the preceding claims the weight ratio of the aqueous phase to the oil phase is 1:2 to 2:1.

10. Kit of parts comprises a cosmetic container, a multi-phase personal care composition according to any one of claims 1 to 9 and instruction for use of the kit.

11. A method for preparing a personal care product comprising the step of shaking a cosmetic container containing composition of any one of claims 1 to 9 by humar hand.

## Patentansprüche

1. Mehrphasige Körperpflegezusammensetzung, umfassend:
(a) eine wässrige Phase, die einen Alkohol in Form eines Diols umfasst; und
(b) eine Ölphase, die flüchtiges Silikon umfasst,
wobei:
i) die wässrige Phase sich von der Ölphase optisch unterscheidet und mit dieser in physikalischem Kontakt steht,
ii) die Menge des Alkohols 2 bis 40 Gew.-% der Zusammensetzung beträgt,
iii) das Gewichtsverhältnis der wässrigen Phase zur Ölphase im Bereich von 1:4 bis 4:1 liegt,
iv) die Zusammensetzung Alkylglucosid umfasst und
v) die Zusammensetzung im Wesentlichen frei von Polysorbat 20 ist, wobei sich "im Wesentlichen frei von" auf eine Menge von 0 bis 0,1 Gew.-% der Zusammensetzung bezieht.

2. Zusammensetzung nach Anspruch 1, wobei der Alkohol 2 bis 10 Kohlenstoffatome aufweist, wobei der Alkohol vorzugsweise Butylenglykol umfasst.

3. Zusammensetzung nach Anspruch 1 oder 2, wobei die Menge des Alkohols 6 bis 30 Gew.-% der Zusammensetzung beträgt.

4. Zusammensetzung nach irgendeinem der vorhergehenden Ansprüche, wobei das flüchtige Silikon 3 bis 10 Siliciumatome enthält, wobei das flüchtige Silikon vorzugsweise Decamethylcyclopentasiloxan ist.

5. Zusammensetzung nach irgendeinem der vorhergehenden Ansprüche, wobei die Menge des flüchtigen Silikons im Bereich von 3 bis 60 Gew.-%, vorzugsweise von 28 bis 42 Gew.-% der Gesamtmenge der Zusammensetzung liegt.

6. Zusammensetzung nach irgendeinem der vorhergehenden Ansprüche, wobei das Gewichtsverhältnis des flüchtigen Silikons zum Alkohol 50:1 bis 1:10, vorzugsweise 6:1 bis 1:1, beträgt.

7. Zusammensetzung nach irgendeinem der vorhergehenden Ansprüche, wobei die Zusammensetzung ein Retinoid, vorzugsweise Retinylpalmitat, Retinylpropionat oder eine Mischung davon, umfasst.

8. Zusammensetzung nach irgendeinem der vorhergehenden Ansprüche, wobei sowohl die wässrige Phase als auch die Ölphase transparent sind.

9. Zusammensetzung nach irgendeinem der vorhergehenden Ansprüche, wobei das Gewichtsverhältnis der wässrigen Phase zur Ölphase 1:2 bis 2:1 beträgt.

10. Kit von Teilen, das einen Kosmetikbehälter, eine mehrphasige Körperpflegezusammensetzung nach irgendeinem der Ansprüche 1 bis 9 und eine Gebrauchsanweisung für das Kit umfasst.

11. Verfahren zur Herstellung eines Körperpflegeprodukts, umfassend den Schritt des Schüttelns eines Kosmetikbehälters, der eine Zusammensetzung nach irgendeinem der Ansprüche 1 bis 9 enthält, mit der Hand.

## Revendications

1. Composition de soin personnel à phases multiples comprenant :
(a) une phase aqueuse comprenant un alcool qui est un diol ; et
(b) une phase huileuse comprenant une silicone volatile,
dans laquelle :
i) la phase aqueuse est visuellement distincte de et en contact physique avec la phase huileuse,
ii) la quantité de l'alcool est de 2 à 40 % en poids de la composition,
iii) le rapport en poids de la phase aqueuse à la phase huileuse est situé dans la plage allant de 1/4 à 4/1,
iv) la composition comprend un alkylglucoside, et
v) la composition est pratiquement exempte de polysorbate 20, dans laquelle "pratiquement exempte de" se réfère à une quantité de 0 à 0,1 % en poids de la composition.

2. Composition selon la revendication 1, dans laquelle l'alcool a 2 à 10 atomes de carbone, de préférence l'alcool comprend du butylèneglycol.

3. Composition selon la revendication 1 ou 2, dans laquelle la quantité de l'alcool est de 6 à 30 % en poids de la composition.

4. Composition selon l'une quelconque des revendications précédentes, dans laquelle la silicone volatile contient 3 à 10 atomes de silicium, de préférence la silicone volatile est le décaméthylcyclopentasiloxane.

5. Composition selon l'une quelconque des revendications précédentes, dans laquelle la quantité de la silicone volatile est située dans la plage allant de 3 à 60 %, de préférence de 28 à 42 % en poids de la quantité totale de la composition.

6. Composition selon l'une quelconque des revendications précédentes, dans laquelle le rapport en poids de la silicone volatile à l'alcool est de 50/1 à 1/10, de préférence de 6/1 à 1/1.

7. Composition selon l'une quelconque des revendications précédentes, dans laquelle la composition comprend un rétinoïde, de préférence le palmitate de rétinyle, le propionate de rétinyle, ou un mélange de ceux-ci.

8. Composition selon l'une quelconque des revendications précédentes, dans laquelle tant la phase aqueuse que la phase huileuse sont transparentes.

9. Composition selon l'une quelconque des revendications précédentes, dans laquelle le rapport en poids de la phase aqueuse à la phase huileuse est de 1/2 à 2/1.

10. Kit de pièces comprenant un récipient à cosmétique, une composition de soin personnel à phases multiples selon l'une quelconque des revendications 1 à 9 et un mode d'emploi du kit.

11. Procédé pour préparer un produit de soin personnel, comprenant l'étape de secouage à la main par un humain d'un récipient à cosmétique contenant la composition de l'une quelconque des revendications 1 à 9.
